# FASCICULE DE BREVET EUROPEEN

(11) **EP 0 684 846 B1**
(45) Date de publication et mention de la délivrance du brevet: **06.05.1999**
(21) Numéro de dépôt: 94905824.2
(22) Date de dépôt: 18.02.1994
(51) Int. Cl.: A61M 5/24, A61M 5/50

(54) **SERINGUE PREREMPLIE DE STOCKAGE ET DE TRANSFERT D'UNE SUBSTANCE MEDICAMENTEUSE LIQUIDE ET STERILE**
VORGEFÜLLTE SPRITZE ZUR BEWAHRUNG UND ABGABE EINES STERILEN FLÜSSIGEN ARZNEIMITTELS
PREFILLED SYRINGE FOR STORING AND DISPENSING A STERILE LIQUID DRUG

(30) Priorité: 19.02.1993 FR 9301998
(43) Date de publication de la demande: 06.12.1995
(73) Titulaire: SANOFI, 75008 Paris (FR)
(72) Inventeur: MEYER, Gabriel, CH-1195 Dully (CH)
(74) Mandataire: Nithardt, Roland
(86) Numéro de dépôt international: IB9400016
(87) Numéro de publication internationale: WO9419034

(56) Documents cités:
- WO-A-87/06141
- WO-A-92/22338
- FR-A- 2 300 578

## Description

La présente invention concerne une seringue préremplie pour le stockage et le transfert d'une substance médicamenteuse liquide et stérile, comportant un réservoir rigide à fond fermé obturé à son extrémité ouverte opposée au fond par un obturateur-piston-vanne agencé pour coulisser axialement, ce réservoir étant mobile axialement à l'intérieur d'un corps cylindrique creux comportant une tige centrale pourvue d'un conduit axial communiquant avec une aiguille fixée à l'extrémité proximale du corps, opposée audit réservoir, cette tige étant pourvue de moyens de liaison agencés pour permettre un accouplement ferme avec ledit obturateur-piston-vanne, ces moyens de liaison comprenant un bloc d'emboîtement terminé par une pointe formée par l'extrémité de la tige et pourvu d'une gorge périphérique agencée pour recevoir une protubérance de l'obturateur-piston-vanne pendant une phase de stockage de la seringue, l'obturateur-piston-vanne comportant un évidement central pour recevoir ce bloc d'emboîtement pendant une phase d'utilisation de la seringue et étant pourvu, dans sa zone centrale et dans le prolongement dudit évidement, d'une fente prédécoupée définie par deux lèvres, et ladite pointe étant pourvue d'une extrémité coupée selon un plan perpendiculaire à son axe et agencée pour écarter lesdites lèvres et ouvrir ladite fente pour mettre en communication ledit conduit axial avec ledit réservoir.

Il existe une grande variété de dispositifs de ce type qui ont tous pour objet de contenir une substance liquide stérile destinée à être transférée par injection directe à un patient ou vers un autre dispositif récepteur.

La seringue préremplie décrite par le brevet américain No 2,574,339 comporte une double aiguille centrale dont une extrémité perfore un piston obturant un conteneur. Cette seringue présente l'avantage de permettre le transfert du liquide contenu dans le conteneur en exerçant une poussée sur ce conteneur sans devoir procéder à d'autres manipulations pour activer la seringue. Cette poussée a pour effet de perforer le piston par l'aiguille centrale pour permettre l'écoulement de la solution à travers cette aiguille. L'inconvénient majeur de cette seringue est qu'il est impossible de l'utiliser pour réaspirer du sang du patient à l'intérieur du conteneur lors d'une injection directe pour effectuer le test "veine", ou un mélange obtenu après transfusion dans un autre dispositif. A cet inconvénient s'ajoutent ceux concernant la fabrication coûteuse d'une double aiguille comportant des biseaux à ses deux extrémités, la perforation par cette aiguille de la partie centrale du piston ce qui génère des particules indésirables qu'il n'est pas possible d'éliminer et finalement le fait que l'aiguille d'injection, fixée irréversiblement au corps de la seringue ne peut pas être escamotée à l'intérieur du corps de cette seringue, après usage. Celle-ci doit être recapuchonnée par l'avant pour la protéger après utilisation ce qui représente aujourd'hui un risque majeur de contamination accidentelle du soignant par piqûre de l'aiguille souillée. La "Food and Drug Administation (FDA)", ainsi que l'American Society of Hospital Pharmacist (ASHP)" encouragent la construction de systèmes dépourvus d'aiguille pour la préparation des solutions injectables et la rétraction à l'intérieur du système de l'aiguille ayant servi à l'injection de cette solution à un patient (Vol. 49, Aug. 1992 Am) Hosp. Pharm. 1851; letter dated October 26, 1992 de Suzan A. Coutrell, Director, ASHP Home Care and Managed Department to Edmund M. Fry, PDA Executive Vice President. En plus cette seringue ne comporte pas de moyens permettant d'assurer sa non-réutilisation. Ceci est vrai tant pour sa partie proximale que pour sa partie distale puisqu'aucun moyen de fixation irréversible du conteneur avec le corps n'est prévu. Par ailleurs, cette seringue est dépourvue de moyens permettant de maintenir la stérilité de la surface du piston devant être perforée par l'aiguille, et de l'aiguille elle-même.

La demande de brevet WO-A-92/18178 décrit également une seringue dont l'obturateur est perforé par une aiguille, ce qui engendre un risque non négligeable d'injection de particules solides dans le circuit sanguin du patient.

La demande de brevet français FR-A- 2 300 578 décrit, dans une des réalisations présentées, un piston-vanne préperforé selon le préambule de la revendication 1. Toutefois cette perforation est réalisée dans une membrane fine et ne permet pas d'assurer une étanchéité absolue pendant le stockage. En outre, la vanne est du type bidirectionnelle réagissant aux pressions différentielles, ce qui crée des problèmes de fonctionnement et des problèmes d'étanchéité.

Le brevet américain US-A-3,841,329 décrit une seringue dans laquelle l'obturateur servant également de piston et de vanne est prépercé. Toutefois, le perçage est fait dans une membrane de très faible épaisseur, étant donné que la fente ainsi réalisée doit s'ouvrir sous l'effet d'une pression exercée par l'intermédiaire du liquide contenu dans le réservoir. De ce fait, ce système présente un défaut d'étanchéité qui implique que l'obturateur-piston-vanne doit être obligatoirement coupé à la tige de transfert avant son positionnement hermétique dans le réservoir et qu'il est impossible d'effectuer une réaspiration, en particulier pour effectuer le test "veine". Le défaut d'étanchéité peut avoir une conséquence plus grave : la perte de la stérilité au stockage.

La seringue décrite par le brevet américain 1,159,663 comporte également une double aiguille. Elle prévoit un couplage par vissage entre le piston et un cylindre central disposé à l'intérieur du corps et entourant la double aiguille. Non seulement cette construction ne résout pas le problème de la réaspiration possible en fin d'utilisation, mais elle apporte des inconvénients supplémentaires. Le conteneur doit être tourné pour activer le système ce qui complique la manipulation et impose que les forces de frottement du piston contre les parois du verte soient toujours supérieures aux forces de vissage. Il convient donc de maîtriser plusieurs paramètres délicats de fabrication et il s'ensuit des déchets onéreux. La fabrication du corps n'est pas évidente par moulage. En effet, il est impossible de réaliser des cycles rapides de production en raison de l'impossibilité de pouvoir refroidir l'intérieur du corps. D'autre part, si dans cette seringue l'air peut être évacué lors du conditionnement par la partie distale du conteneur, ce qui présente par ailleurs un désavantage au niveau de la production dudit conteneur ayant avantageusement un fond fermé à la flamme, ce dernier contient obligatoirement une certaine quantité de gaz qu'il sera nécessaire de purger avant de procéder à une injection directe au patient.

Pour répondre aux exigences actuelles de l'industrie pharmaceutique, une seringue préremplie ne doit pas présenter de tels inconvénients. Pour répondre à ces exigences, il serait nécessaire de développer des moyens permettant de conditionner une substance liquide dans un conteneur en verre à fond fermé à la flamme en l'absence totale de gaz afin d'éviter l'opération de purge et d'améliorer la conservation des solutions ne supportant pas la présence d'oxygène ou lorsque le test "veine" n'est pas requis, comme cela peut être le cas dans certains pays pour les injections sous-cutanées de petits volumes. Il est d'ailleurs impossible d'éliminer complètement l'oxygène même en procédant à un gazage méticuleux au moyen d'un gaz neutre tel que l'azote ou le gaz carbonique tant qu'il subsiste un résidu gazeux dans le réservoir.

Les dispositifs proposés dans les brevets américains 3,563,415 et 3,722,512 permettent de supprimer toute présence de gaz surmontant la solution liquide en proposant un long piston comportant un canal central non obturé du côté de la substance médicamenteuse à conserver. Cette construction ne permet pas d'obturer de manière étanche le conteneur après le conditionnement de la solution en vue de le soumettre à un autoclavage final que les autorités d'enregistrement exigent actuellement pour toutes les substances liquides supportant le traitement à la chaleur.

Les dispositifs proposés dans les brevets européens publiés sous les No. 0 111 796 et 0 146 558 décrivent des systèmes similaires qui ne résolvent pas les problèmes posés par la présence de gaz dans un conteneur à fond fermé, ni celui de l'autoclavage du conteneur de manière indépendante du corps. Toutefois ces dispositifs permettent de supprimer la perforation du piston en proposant un système de piston jouant un rôle de vanne en coopérant avec une forme appropriée du col du conteneur. Cette construction oblige l'utilisateur à orienter la seringue vers le haut et à pousser le conteneur d'une longueur précise pour amener le canal radial de l'obturateur en communication avec le grand diamètre du conteneur afin, dans un premier temps, de purger l'air sans projeter du liquide à l'extérieur. Ces manipulations sont délicates et incompatibles avec un dispositif moderne d'utilisation instinctive et sans risque d'erreur. De tels dispositifs doivent obligatoirement être utilisés avec des accessoires qui permettent de limiter les courses d'activation et de purge de manière précise. En plus ces systèmes ne peuvent pas être utilisés de manière aisée et fiable en vue de transférer leur contenu dans un flacon contenant une autre substance, et ils ne permettent pas la réaspiration complète du mélange reconstitué à l'intérieur du conteneur sans aspirer un volume de gaz plus moins important qu'il faudra par la suite purger à travers le canal central qui contiendra obligatoirement un certain volume de solution. De tels dispositifs devraient être capables de réaspirer le mélange intégralement et de manière contrôlée afin de procéder à une injection sans obligation de purge.

La seringue proposée dans le brevet allemand No 22 62 706 ne résout pas les problèmes énoncés ci-dessus et présente les mêmes inconvénients que ceux mentionnés en relation avec le brevet américain 1,159,663.

On constate donc que tous les dispositifs de l'art antérieur présentent une série de désavantages et que certains, s'ils résolvent en partie seulement l'un ou l'autre des inconvénients, sont basés sur des constructions qui présentent des désavantages supplémentaires allant du simple désagrément au risque médical d'injecter de l'air au patient ou à l'impossibilité de conserver de manière adéquate la solution stérilement.

En outre, tous ces dispositifs connus ne permettent pas de fixer de manière stable, par une connexion adéquate, les seringues préremplies à des flacons normalisés contenant une substance sèche prévue pour être mélangée à un solvant conditionné dans ces seringues, ni de protéger l'aiguille lors de la perforation du bouchon obturant le flacon contenant l'autre substance afin de préserver le biseau pour procéder à une injection ultérieure du mélange reconstitué et ceci afin de correspondre aux récentes recommandations ci-dessus de la FDA et ASHP. Enfin ces seringues connues ne permettent pas de reconstituer des substances à forte activité pharmacologique telles que les substances anti-cancer et antibiotiques, dont certaines peuvent dégager des gaz lors de la reconstitution. En effet, pour ces substances, il est impératif que le dispositif de stockage et de transfert soit équipé de moyens permettant la connexion étanche avec le flacon contenant ces substances et que, lors de la déconnexion du dispositif de transfert, il ne se produise aucune nébulisation. Dans certaines circonstances, il est nécessaire de ponctionner le mélange contenu dans au moins deux flacons à l'intérieur de la seringue afin d'adapter la posologie en fonction du poids du patient, de son âge et de l'intensité du traitement. Toutes ces opérations doivent pouvoir se faire sans danger de contamination du soignant ou de son environnement et sans contamination du mélange par le milieu ambiant ou par d'autres substances.

Lorsque ces seringues sont déjà équipées d'une aiguille d'injection, celle-ci doit être protégée du milieu ambiant et de toute autre souillure afin qu'elle reste intacte jusqu'au moment de l'injection et surtout afin d'éviter les piqûres accidentelles lors de la préparation où une aiguille ne devrait pas être utilisée, selon les récentes recommandations de la FDA et de l'ASHP. En plus des moyens doivent être prévus pour éviter les piqûres accidentelles au cours de toutes les opérations de transfert, ainsi qu'après l'utilisation de la seringue pour une injection.

On notera que les dispositifs proposés ne peuvent pas être utilisés comme distributeurs de gouttes ophtalmiques stériles en raison de l'absence de moyens d'obturation du piston du conteneur en fin d'utilisation ou comme pulvérisateurs nasaux, car aucun moyen n'est prévu pour que le piston s'ouvre à une pression déterminée essentielle pour obtenir une nébulisation qui ne peut être atteinte qu'à un certain seuil de pression. Ils ne permettent pas non plus l'administration de doses successives précises car aucun moyen n'est prévu pour équiper ces dispositifs de limiteurs de course. En plus, lorsque ces dispositifs ophtalmiques et nasaux sont utilisés pour des doses multiples, aucun moyen n'est prévu pour inhiber la croissance des bactéries dans l'embout applicateur.

La demande internationale de brevet publiée sous le No WO 91/00215 prévoit un dispositif permettant de protéger l'aiguille lors d'un transfert de solution dans un flacon normalisé. Toutefois ce dispositif est indépendant de telle sorte que la disponibilité d'un tel dispositif n'est pas garantie au moment du transfert. De tels moyens doivent impérativement faire partie d'un dispositif de stockage et de transfert prêt à l'emploi pour respecter les exigences de la FDA et de l'ASHP.

Des dispositifs prévoyant la protection de l'aiguille après utilisation, par rétraction de l'aiguille dans un organe protecteur ou par l'avance d'un organe protecteur par-dessus l'aiguille, sont proposés dans plusieurs brevets. Toutes les constructions proposées font appel à des seringues obturées à leur extrémité distale par un piston dont la tige actionne son coulissement. Aucun moyen de ce type n'a été prévu sur un dispositif prérempli comportant un conteneur à fond fermé, notamment dans les dispositifs objets des brevets US-5,098,403, US-5,084,018, F-2 667 249, ES-2 023 772 et US-5,114,410.

La présente invention se propose de pallier tous les inconvénients et lacunes énumérés ci-dessus en proposant une seringue qui solutionne l'ensemble des problèmes inhérents aux seringues connues et qui répond aux exigences les plus récentes d'un dispositif de stockage et de transfert d'une solution liquide stérile pour toute sortes d'applications telles que l'injection directe à un patient au moyen d'une aiguille, la pulvérisation nasale stérile et la distribution de gouttes ophtalmiques stériles.

Ce but est atteint par la seringue selon l'invention, caractérisée en ce que ledit bloc d'emboîtement comporte une partie étroite pourvue de deux bourrelets disposés de part et d'autre de la gorge périphérique, ledit premier bourrelet étant agencé pour offrir une résistance à la compression de la protubérance inférieure à celle du deuxième bourrelet.

Selon un mode de réalisation avantageux, ladite tige est solidaire d'une embase agencée pour prendre appui contre une surface intérieure de l'extrémité distale du corps cylindrique creux.

D'une manière préférentielle, ledit bloc d'emboîtement comporte une partie large dont le diamètre est sensiblement égal au diamètre intérieur du corps cylindrique creux pour assurer le centrage de ladite tige à l'intérieur dudit corps.

De façon avantageuse, ledit premier bourrelet a une section inférieure à celle du deuxième bourrelet.

Dans la forme de réalisation préférée, le réservoir comporte un bourrelet extérieur agencé pour coopérer avec une gorge ménagée à l'extrémité libre du corps et un bourrelet intérieur agencé pour coopérer avec un renflement situé à la base de la tige en fin d'utilisation.

Ladite fente prédécoupée est avantageusement centrée par rapport audit évidement central de l'obturateur-piston-vanne

Dans toutes les formes de réalisation, le diamètre de la pointe formant l'extrémité du bloc d'emboîtement est inférieur au diamètre de la zone centrale de l'obturateur-piston-vanne.

La présente invention et ses principaux avantages seront mieux compris en référence à la description d'exemples de réalisation non limitatifs, illustrés par les dessins annexés dans lesquels :
la figure 1 représente une vue en coupe axiale de la seringue préremplie selon l'invention, illustrée dans sa position de stockage,
la figure 2 représente la seringue de la figure 1 à la fin de la phase d'injection,
la figure 3 montre la seringue de la figure 1 avec l'aiguille neutralisée en fin d'utilisation,
la figure 4 représente une vue agrandie de l'obturateur-piston-vanne de la seringue selon l'invention pendant le stockage,
la figure 5 représente une vue similaire à celle de la figure 4 de la seringue selon l'invention pendant l'utilisation,
la figure 6 représente une vue en coupe axiale illustrant un outillage pour percer l'obturateur-piston-vanne,
les figures 7 à 12 illustrent le procédé de remplissage et de contrôle du réservoir de la seringue selon l'invention,
les figures 13 à 15 représentent les phases d'assemblage, de contrôle et d'étiquetage des deux sous-ensembles constituant la seringue de l'invention,
les figures 16 et 17 illustrent deux phases d' utilisation de la seringue de l'invention,
les figures 18 à 21 sont des vues représentant une variante de réalisation de la seringue de l'invention dans différentes phases de son utilisation,
la figure 22 illustre une seringue contenant une substance solide ou pâteuse,
la figure 23 représente la phase de dilution ou dissolution de la substance contenue dans la seringue de la figure 22, et
la figure 24 illustre une variante de réalisation d'une seringue selon l'invention.

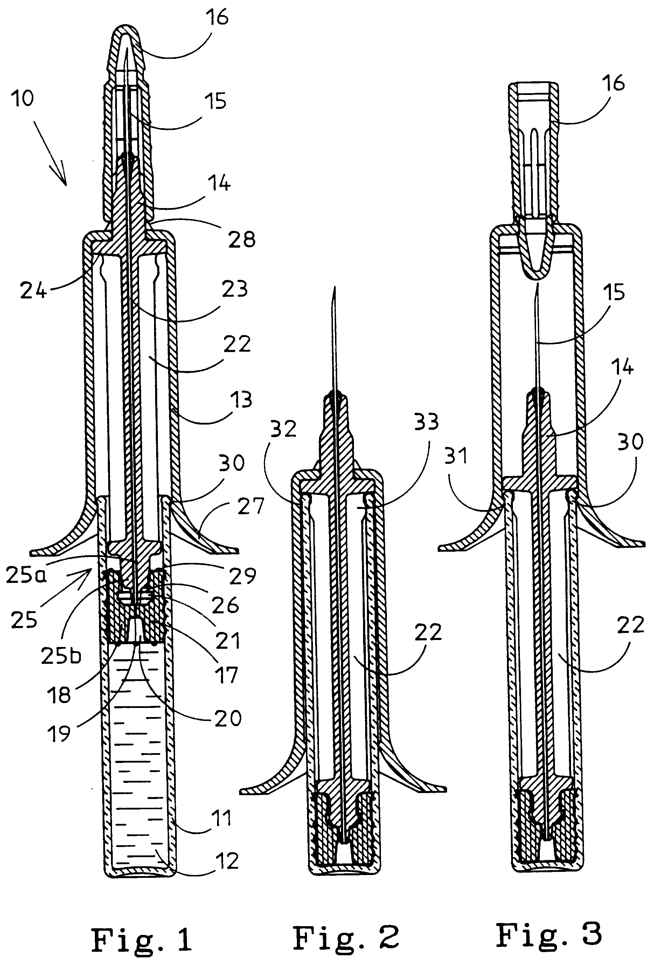

En référence à la figure 1, la seringue préremplie 10 comporte un réservoir 11 rigide, de préférence en verre pour pouvoir supporter les contraintes thermiques d'une stérilisation par autoclavage ou similaire, rempli d'une substance médicinale injectable 12 et engagé dans l'ouverture proximale d'un corps 13 dont l' extrémité distale est équipée d'un embout 14 portant une aiguille 15 protégée par un capuchon 16. Le réservoir est obturé par un obturateur-piston-vanne 17 ayant une section dont le profil présente un aspect de H et pourvu d'une zone centrale 18 comportant une fente 19 qui relie deux évidements respectivement amont 20 et aval 21. L' évidement amont est disposé du côté du réservoir et l' évidement aval est disposé du côté du corps 13. A l'intérieur de ce corps est montée une tige 22 rigide dans laquelle est ménagé un conduit axial 23. Dans la construction représentée, la tige 22 est solidaire d'une embase 24 elle-même solidaire de l' embout 14. Cette tige est complétée à son extrémité opposée à l'embase 24 par un bloc d'emboîtement 25 agencé pour s'engager dans l' évidement aval 21 de l' obturateur-piston-vanne 17. Dans cette réalisation également, le conduit axial 23 est situé dans le prolongement de l'aiguille 15 ou par un canal ménagé à l'intérieur de la tige 22. Le bloc d'emboîtement 25 comporte à son extrémité une pointe 26 entourant l'extrémité de la tige 22 ou constituée par cette extrémité proprement dite, cette pointe étant agencée pour pénétrer dans la fente 19 et pour l'ouvrir en écartant les lèvres qui la délimitent au moment où le bloc d'emboîtement s'engage partiellement dans l'évidement aval 21 de l'obturateur-piston-vanne.

Par ailleurs, le corps 13 est pourvu d'ailettes 27 réalisées de préférence d'une pièce avec ce corps par une technique de moulage par injection en matière synthétique. On notera que l'aiguille 15 et la tige pourraient être réalisées d'une pièce, mais pour des raisons de coût de production, il est préférable d'utiliser une aiguille standard et de la fixer par collage, soudure ou surmoulage à la tige, à l'extrémité de l'embout 14. Enfin, des bras de liaison 28 assurent la liaison du capuchon 16 avec le corps 13.

Dans la réalisation représentée, le bloc d'emboîtement 25 comporte une partie large 25a, dont le diamètre est sensiblement égal au diamètre intérieur du corps, et une partie étroite 25b qui correspond au diamètre de l' évidement aval 21. La partie large assure le centrage de la tige, et la partie étroite assure l' accouplement de l' obturateur-piston-vanne 17 avec la tige 22. Pendant le stockage, l'obturateur-piston-vanne est pré-accouplé à la tige par friction pour garantir le maintien de la stérilité au niveau de l'évidement aval 21. Il joue effectivement le rôle d'un obturateur et ferme le réservoir 11 pendant cette phase de stockage. A partir du moment où le couplage de l'obturateur-piston-vanne et de la tige est effectué complètement, cet organe devient un piston qui refoule la substance médicinale 12 liquide à travers la fente 19 maintenue ouverte par la pointe 26, dans le conduit axial 23 en direction de l'aiguille 15.

Il est à remarquer que la fente 19 n'est pas perforée par la pointe 26 mais qu'elle est prédécoupée, c'est-à-dire réalisée au moment de la fabrication de l'obturateur-piston-vanne. Cette pointe 26 écarte par déformation élastique les lèvres de la fente 19. Son ouverture s'effectue donc sans enlèvement de matière, ce qui supprime le risque d'injecter des particules dans l'organisme du patient. Cette fente s'ouvre lorsque la pointe s'y engage et s'obture dès qu'elle est retirée. De ce fait, elle remplit les fonctions d'une vanne.

L' évidement aval 21 est entouré d'un bourrelet 29 qui crée une zone de friction avec la partie étroite 25b du bloc d'emboîtement 25 constituant la tête de la tige 22. Cette zone de friction garantit l'étanchéité bactérienne de la zone inférieure de l'évidement aval 21 pendant le stockage de la seringue.

De cette manière, la fente reste stérile pendant le stockage. L'embase 24 et l'embout 14 constituent une autre barrière bactérienne. Cette barrière est complétée par le capuchon dont la base est fixée par friction sur l'embout 14.

L'extrémité ouverte du réservoir comporte un bourrelet extérieur 30 qui coopère avec une gorge appropriée 31 ménagée à l'extrémité libre du corps 13 pour assurer un couplage irréversible du réservoir prérempli, équipé de son obturateur-piston-vanne 17, sur le corps.

La seringue représentée est prête à l'emploi et ne contient aucun gaz, ce qui évite une opération de purge. De ce fait, l'aiguille reste sèche et non contaminée par un écoulement de substance médicinale entraînée par le gaz au moment du dégazage des seringues classiques, jusqu'au moment de l'injection.

Lors de la fabrication de cette seringue, le réservoir subit un lavage, une décontamination par ultra-sons, un siliconage adéquat des parois internes, et une fixation du film par réticulation à la chaleur pour former une pellicule hydrophobe ayant des caractéristiques de glissement idéales. En même temps que s'opère la réticulation, le réservoir est dépyrogénisé. Ces deux dernières opérations, effectuées à une température avoisinant 300°C pendant vingt minutes, sont rendues possibles par le fait que l'aiguille n'est pas fixée par collage au réservoir. Le réservoir est ensuite rempli et l'obturateur est mis en place. Grâce à la fente centrale de l' obturateur, la tige de positionnement du piston d'un automate de montage mettant cet obturateur en place communique, grâce à un canal central, avec des moyens accessoires d'aspiration. Cette tige creuse, appuyant sur la partie centrale fendue, exerce une déformation élastique des parois et la fente devient un orifice ouvert permettant au gaz surmontant la solution de s'échapper axialement par cette fente. Dans certains cas, un très faible volume de la solution peut également passer par cet orifice. Ce faible volume est aspiré dans la tige centrale et récupéré dans un récipient clos, grâce aux moyens accessoires d'aspiration de l'automate de montage. Ensuite, la fente 19 se referme et l'obturateur-piston-vanne retrouve sa fonction d'obturateur étanche.

Ainsi ce processus permet de conditionner une solution stérile dans un conteneur en verre cylindrique à fond fermé, par exemple scellé à la flamme, en l'absence totale de gaz grâce à la construction de l'obturateur-piston-vanne.

Après ces opérations le réservoir est prêt à subir un autoclavage final lorsque la solution supporte une température de 120°C pendant vingt minutes. L'autoclave doit être du type à contrepression pour empêcher tout mouvement de l'obturateur et préserver l'auto-obturation de la fente.

Les autres composants de la seringue définissent un injecteur. Cet injecteur est prémonté et destiné à être accouplé au réservoir. Il peut être préalablement stérilisé par autoclavage ou radiostérilise. Toutefois, il pourrait ne pas être totalement stérile et être assemblé avec le réservoir. Dans ce cas, l'autoclavage final s'effectue sur le produit fini dans les mêmes conditions. Ce processus de conditionnement est valable pour l'ensemble des dispositifs à composant liquide.

La figure 2 montre la seringue en fin d'injection. Pour injecter la substance médicinale, il faut retirer le capuchon 16 en brisant les bras de liaison 28, puis, sans autre démarche, l' opérateur pique le patient et exerce ensuite une poussée axiale sur le fond du réservoir, ce qui a pour effet d'augmenter la pression dans la solution jusqu'à un seuil déterminé, à partir duquel la tige pénètre dans la fente pour ouvrir la voie permettant à la substance de s'écouler à travers le conduit vers les tissus du patient perforés par l'aiguille reliée à ce conduit.

Lorsque le réservoir est complètement vide, un bourrelet intérieur 32 ménagé à l'extrémité ouverte de ce réservoir coopère avec un renflement 33 situé à la base de la tige 22 pour solidariser la tige avec le réservoir et empêcher une réutilisation de la seringue.

La figure 3 montre que, grâce à cette solidarisation, la tige 22 est entraînée en même temps que le réservoir vers l'extrémité ouverte du corps, si on retire ce réservoir. L'aiguille, qui est solidaire de cette tige, est escamotée à l'intérieur du corps de la seringue jusqu'à une position arrière dans laquelle le bourrelet extérieur 30 coopère avec la gorge 31. De cette manière, on bloque l'aiguille en position protégée par le corps. Accessoirement, comme le montre la figure, la pointe du capuchon protecteur 16 peut être du type "flèche" pour s'enclipser irréversiblement lorsque ledit capuchon a été retourné dans l'orifice du corps de la seringue ouvert par l'embout 14 lorsque l'aiguille a été escamotée. Cette dernière construction permet de neutraliser formellement la seringue après usage et d'interdire l'accès à la pointe souillée de l'aiguille. Il faut noter que l'escamotage de l'aiguille se fait depuis l'arrière de la seringue. Lorsque l'aiguille est escamotée à l'intérieur du corps, les risques de piqûres accidentelles sont nuls. L'aiguille n'étant plus apparente, il n'y a plus de risque lorsque l'on place le capuchon dans l'ouverture du corps pour bloquer l'accès à l'aiguille.

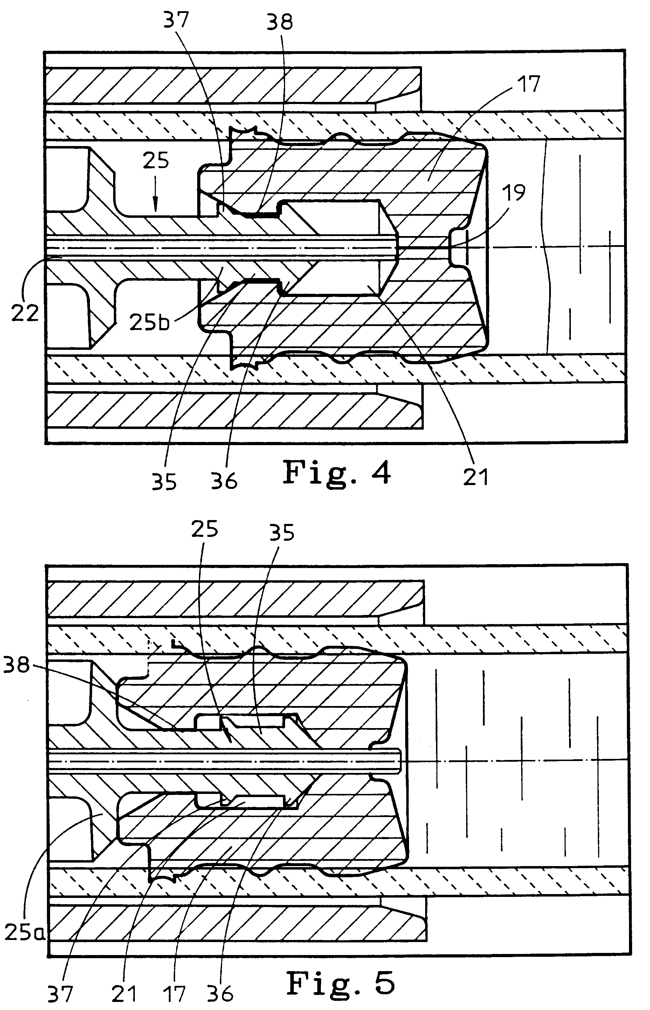

Les figures 4 et 5 représentent des vues agrandies de l'obturateur-piston-vanne 17, dont la construction est sensiblement différente de celle de l'obturateur-piston-vanne des figures précédentes. On notera que le bloc d'emboîtement 25 comporte une gorge périphérique 35 délimitée par deux bourrelets rigides 36 et 37 disposés de part et d'autre de ladite gorge. Cette gorge est agencée pour recevoir une protubérance annulaire 38 ménagée dans la zone centrale, dans l'évidement aval 21 de l'obturateur-piston-vanne. La protubérance 38 est engagée, pendant le stockage représenté par la figure 4, dans la gorge périphérique 35. Au moment de la mise en place du bloc d'emboîtement dans l'évidement aval de l'obturateur-piston-vanne, qui peut être réalisée au moyen d'un organe de poussée équipé d'un ressort taré, le premier bourrelet 36 comprime la matière élastique de la protubérance 38 de l'obturateur-piston-vanne 17 pour la dépasser. Le deuxième bourrelet 37 arrive en butée contre cette protubérance lorsque le premier bourrelet 36 est dégagé pour venir en appui contre le flanc antérieur de la protubérance 38. A cet effet, la résistance opposée au bourrelet 36 doit être inférieure à celle opposée au bourrelet 37. Dans ce but, le bourrelet 36 a de préférence une section inférieure à celle du bourrelet 37. L'extrémité de la tige 22 est en appui contre la fente 19, sans toutefois s'y engager.

En revanche, dans la position représentée par la figure 5, la tige 22 est engagée dans la fente. La pression exercée par la fente sur les lèvres qui la délimitent a provoqué son ouverture. Le bloc d'emboîtement 25 est entièrement logé dans l'évidement aval 21 et la partie large 25a de ce bloc est en appui contre l' obturateur-piston-vanne.

Au stockage, l' étanchéité et la préservation de la stérilité sont assurées par des barrières anti-bactériennes constituées par les lèvres de la fente 19 et par la protubérance 38 et les épaulements 36 et 37. Le bourrelet 36 a une résistance inférieure à celle du bourrelet 37 au passage de la protubérance 38, ce qui permet d'effectuer un montage parfait du réservoir grâce, par exemple, à la poussée exercée par un ressort taré. L'étanchéité obtenue à ce niveau peut être vérifiée au "bain bleu" en dépression et en surpression de ± 0,25 bar pendant 30 minutes, selon les normes de la pharmacopée. La hauteur de la fente 19 est relativement importante, de même que la compression exercée par les lèvres. Ceci est rendu possible par le fait que l'ouverture s'effectue par l'exercice d'une poussée mécanique par l'extrémité de la tige et non par une surpression exercée par un liquide. La résistance au test du "bain bleu" est une démonstration de l'efficacité du système. Aucun autre système antérieur ne satisfait à ce test.

La réaspiration est possible avec cette seringue vu que la fente est maintenue ouverte tant que la tige y est introduite et que le bourrelet 37 prend appui contre la protubérance 38. Tout déplacement relatif de l'obturateur-piston-vanne et du réservoir provoque soit une éjection soit une aspiration, selon le sens du déplacement.

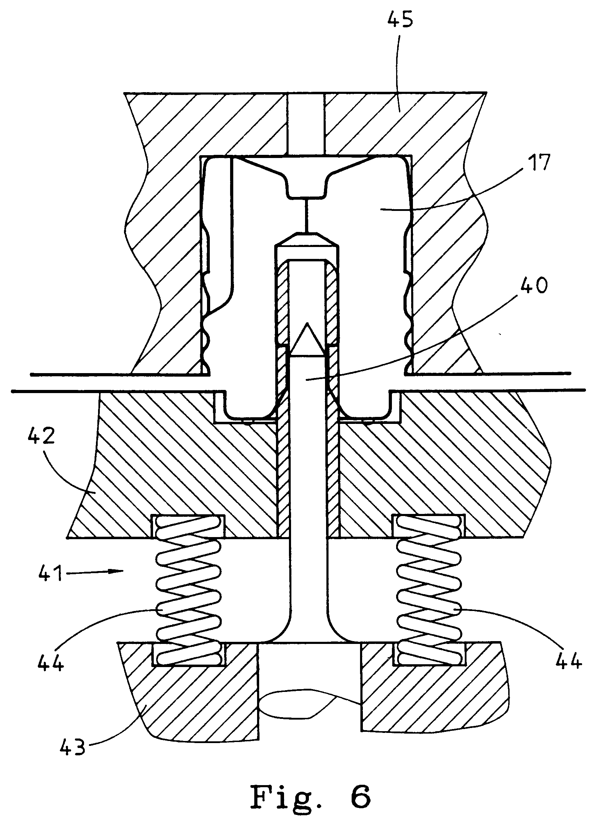

La figure 6 illustre un mode de réalisation d'une fente 19 parfaitement centrée. Une tige de perçage montée sur un outil 41 composée de deux pièces 42 et 43 assemblées par des ressorts 44 assure le perçage de l'obturateur-piston-vanne 17, maintenu en place dans une cavité appropriée d'un support 45.

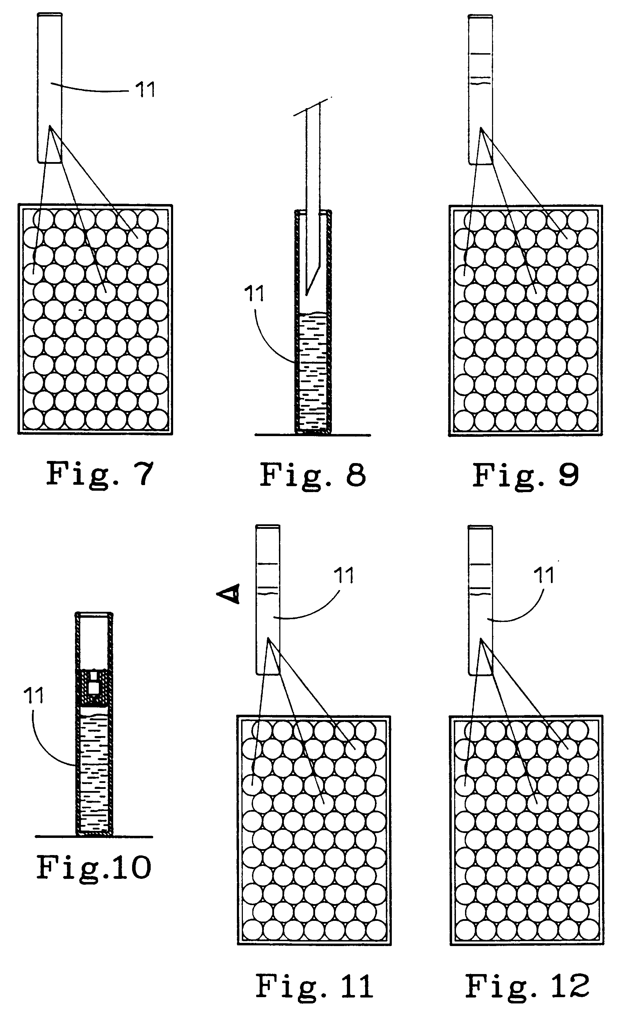

Les figures 7 à 12 illustrent les phases de lavage-siliconage-stérilisation (fig. 7), remplissage (fig. 8) mise en magasin pour transfert (fig. 9), obturation (fig. 10), contrôle visuel (fig. 11) et mise en magasin (fig. 12) du réservoir 11, pour autoclavage en surpression.

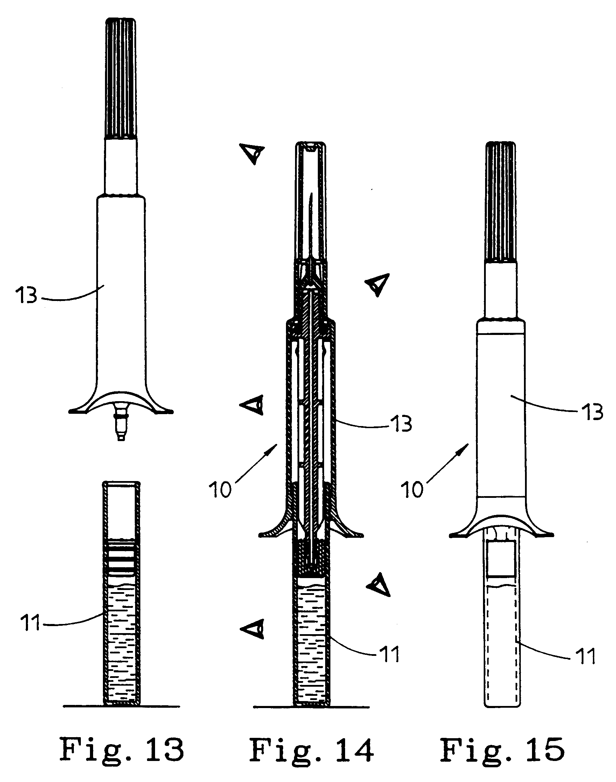

La figure 13 illustre l'assemblage des deux sous-ensembles, respectivement le réservoir 11 et le corps 13, constituant la seringue 10.

La figure 14 illustre la phase de contrôle final de la seringue 10 et la figure 15 représente l'étiquetage.

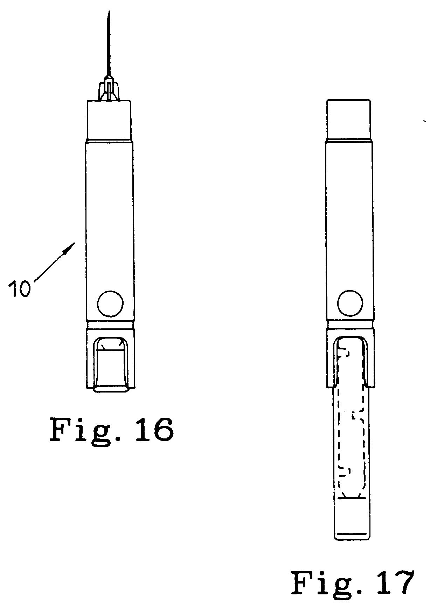

La figure 16 représente la seringue en fin d'injection et la figure 17 la montre lorsque l'aiguille a été rétractée.

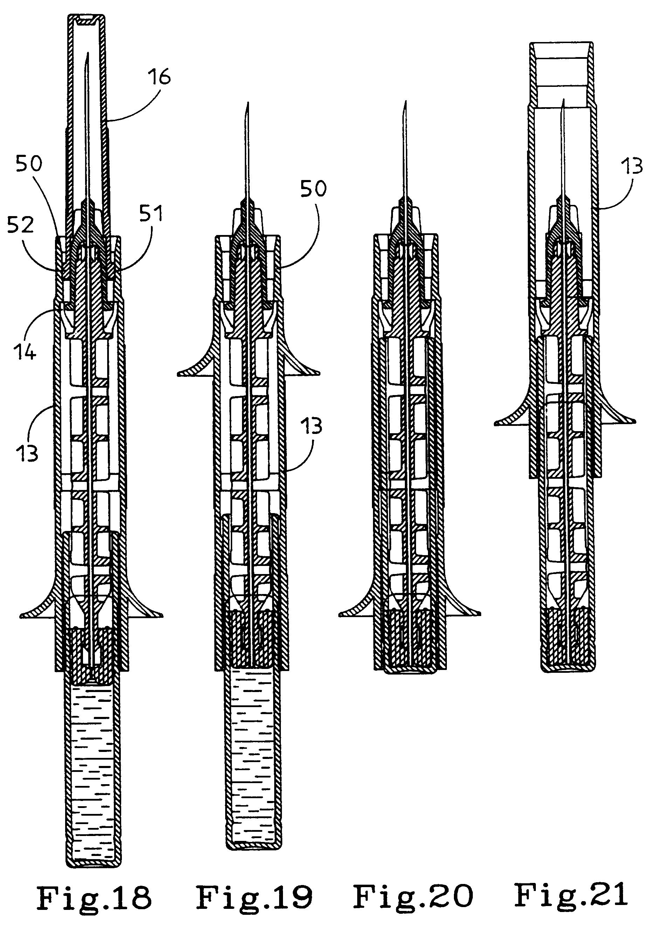

Les figures 18 à 21 représentent une autre forme de réalisation de la seringue, respectivement au stockage (fig. 18), en utilisation (fig. 19), en fin d'injection (fig. 20) et après la rétraction de l'aiguille (fig. 21). On notera que le corps 13 est pourvu d'une jupe 50 qui se prolonge jusqu'au-delà de l'embout porte-aiguille 14. Une zone d'étanchéité 51 est définie par l'appui d'un bourrelet 52 ménagé à la base du capuchon 16 de protection de l'aiguille, contre la jupe 50.

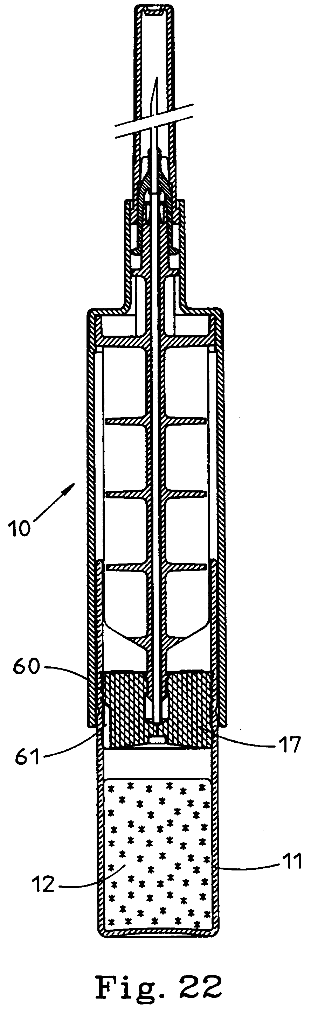

La figure 22 décrit une seringue 10 dont le réservoir 11 contient une substance 12 sèche qui peut être une poudre, un lyophilisat, ou visqueuse nécessitant une dilution avant son injection.

L'obturateur-piston-vanne 17 comporte au moins un bourrelet 60 qui assure une étanchéité parfaite, ce bourrelet étant justifié lorsque la substance est stockée sous vide partiel ou absorbe de l'anhydride carbonique pendant le stockage, pour prendre appui contre un bourrelet interne 61 du réservoir 11.

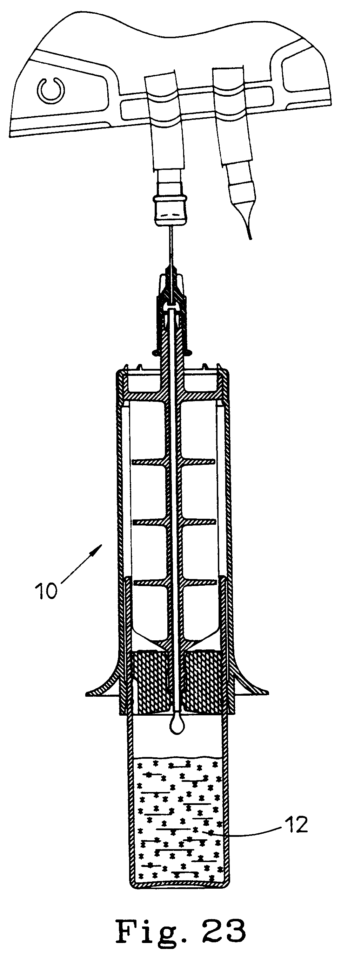

La figure 23 illustre la seringue 10 connectée à une poche contenant un diluant ou un solvant destiné à diluer ou dissoudre la substance 12. Après cette dilution ou dissolution, le contenu peut être transféré dans la poche, dans une autre poche, une bouteille ou similaire.

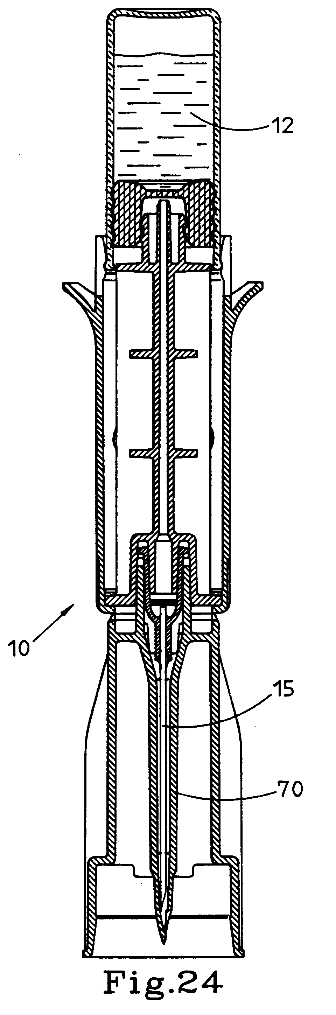

La figure 24 représente une seringue 10 équipée d'une aiguille 15. Cette aiguille est initialement protégée par un dispositif perforateur 70 destiné à percer le bouchon d'obturation d'un flacon pour permettre le mélange de la substance 12 avec un composant provenant d'un autre conteneur. Après le transfert de ce composant dans le réservoir, le dispositif perforateur est séparé de la seringue qui devient une seringue identique ou similaire à celle de la figure 1.

## Revendications

1. Seringue préremplie pour le stockage et le transfert d'une substance médicamenteuse liquide et stérile, comportant un réservoir (11) rigide à fond fermé obturé à son extrémité ouverte opposée au fond par un obturateur-piston-vanne (17) agencé pour coulisser axialement, ce réservoir étant mobile axialement à l'intérieur d'un corps cylindrique creux (13) comportant une tige centrale (22) pourvue d'un conduit axial (23) communiquant avec une aiguille (15) fixée à l'extrémité proximale du corps, opposée audit réservoir, cette tige étant pourvue de moyens de liaison agencés pour permettre un accouplement ferme avec ledit obturateur-piston-vanne, ces moyens de liaison comprenant un bloc d'emboîtement (25) terminé par une pointe (26) formée par l'extrémité de la tige (22) et pourvu d'une gorge périphérique (35) agencée pour recevoir une protubérance (38) de l'obturateur-piston-vanne pendant une phase de stockage de la seringue, l'obturateur-piston-vanne comportant un évidement central (21) pour recevoir ce bloc d'emboîtement pendant une phase d'utilisation de la seringue et étant pourvu, dans sa zone centrale (18) et dans le prolongement dudit évidement (21), d'une fente prédécoupée (19) définie par deux lèvres, et ladite pointe (26) étant pourvue d'une extrémité coupée selon un plan perpendiculaire à son axe et agencée pour écarter lesdites lèvres et ouvrir ladite fente pour mettre en communication ledit conduit axial (23) avec ledit réservoir (11), le diamètre de la pointe (26) est inférieur au diamètre de la zone centrale (18) de l'obturateur-piston-vanne (17), caractérisée en ce que ledit bloc d'emboîtement (25) comporte une partie étroite (25b) pourvue de deux bourrelets (36) et (37) disposés de part et d'autre de la gorge périphérique (35), ledit premier bourrelet (36) étant agencé pour offrir une résistance à la compression de la protubérance (38) inférieure à celle du deuxième bourrelet (37).

2. Seringue selon la revendication 1, caractérisée en ce que ladite tige (22) est solidaire d'une embase (24) agencée pour prendre appui contre une surface intérieure de l'extrémité distale du corps cylindrique creux (13).

3. Seringue selon la revendication 1, caractérisée en ce que ledit bloc d'emboîtement (25) comporte une partie large (25a) dont le diamètre est sensiblement égal au diamètre intérieur du corps cylindrique creux (13) pour assurer le centrage de la tige (22) à l'intérieur dudit corps.

4. Seringue selon la revendication 1, caractérisée en ce que ledit premier bourrelet (36) a une section inférieure à celle du deuxième bourrelet (37).

5. Seringue selon la revendication 1, caractérisée en ce que le réservoir (11) comporte un bourrelet extérieur (30) agencé pour coopérer avec une gorge (31) ménagée à l'extrémité libre du corps (13).

6. Seringue selon la revendication 1, caractérisée en ce que le réservoir (11) comporte un bourrelet intérieur (32) agencé pour coopérer avec un renflement (33) situé à la base de la tige (22) en fin d'utilisation.

7. Seringue selon la revendication 1, caractérisée en ce que ladite fente prédécoupée (19) est centrée par rapport audit évidement central (21) de l'obturateur-piston-vanne (17).

## Claims

1. Prefilled syringe for storing and transferring of a sterile medical substance comprising a rigid reservoir (11) having a closed bottom and being closed at its open end opposite to the bottom by a stopper-piston-valve (17) which is axially slidable, this reservoir being axially movable within a hollow cylindrical body (13) comprising a central shaft (22) provided with an axial conduit (23) which communicates with a needle (15) fixed to the proximal end of the body, opposite to said reservoir, this shaft being provided with connecting means adapted to allow firm connection to said stopper-piston-valve, said connecting means including a clamping block (25) ended with a tip (26) formed by the end of the shaft (22) and provided with a peripheral neck (35) adapted to receive a protuberance (38) of the stopper-piston-valve during a phase of storage of the syringe, the stopper-piston-valve comprising a central cavity (21) for receiving this clamping block during a phase of use of the syringe and being provided with a pre-cut slot (19) defined by two lips and disposed in its central zone (18) and in prolongation of said central cavity (21), and said tip (26) being provided with one end cut off along a plan perpendicular to its axis and adapted to separate said lips and to open said slot so as to allow communication between said axial conduit (23) and said reservoir (11), the diameter of the tip (26) being inferior to the diameter of the central zone (18) of the stopper-piston-valve (17), characterized in that said clamping block (25) comprises a narrow portion (25b) provided with two shoulders (36) and (37) disposed on both sides of the peripheral neck (35), said first shoulder (36) being adapted to offer less resistance to compression provided by the protuberance (38) than that of the second shoulder (37)

2. Syringe according to claim 1, characterized in that said shaft (22) is integral with a flange (24) adapted to bear on an inner surface of the distal end of the hollow cylindrical body (13).

3. Syringe according to claim 1, characterized in that said clamping block (25) has a wide portion (25a) with a diameter which is substantially equal to the inner diameter of the hollow cylindrical body (13) to ensure centering of the shaft (22) within said body.

4. Syringe according to claim 1, characterized in that said first shoulder (36) has a smaller cross-section than the second shoulder (37).

5. Syringe according to claim 1, characterized in that the reservoir (11) comprises an external shoulder (30) adapted to cooperate with a neck (31) provided at the free end of the body (13).

6. Syringe according to claim 1, characterized in that the reservoir (11) comprises an internal shoulder (32) adapted to co-operate with an enlargement (33) located at the base of the shaft (22) at the end of said phase of use.

7. Syringe according to claim 1, characterized in that said pre-cut slot (19) is centered with respect to said central cavity (21) of the stopper-piston-valve (17).

## Patentansprüche

1. Vorgefüllte Spritze für die Lagerung und die Übertragung einer flüssigen und sterilen medizinischen Substanz, die ein starres, mit einem geschlossenen Boden versehenes Reservoir (11) aufweist, das an seinem offenen, dem Boden gegenüberliegenden Ende mittels einer axial gleitbar angeordneten Kolben-Ventil-Verschlußvorrichtung (17) abgedichtet ist, wobei das Reservoir im Inneren eines zylindrischen Hohlkörpers (13) axial bewegbar ist, der eine mittige Stange (22) aufweist, die mit einer Axialleitung (23) versehen ist, die mit einer Nadel (15) in Verbindung steht, die am nächstliegenden Ende des Körpers fixiert ist, das dem besagten Reservoir gegenüberliegt, wobei die Stange mit Verbindungsmitteln versehen ist, die so angeordnet sind, daß sie eine feste Koppelung mit der besagten Kolben-Ventil-Verschlußvorrichtung ermöglichen, wobei die Verbindungsmittel einen Einlassungsblock (25) aufweisen, der durch eine durch das Ende der Stange (22) gebildete Spitze (26) abgeschlossen ist und mit einer Umfangsrille (35) versehen ist, die so angeordnet ist, daß sie eine Ausstülpung (38) der Kolben-Ventil-Verschlußvorrichtung während einer Lagerungsphase der Spritze aufnimmt, wobei die Kolben-Ventil-Verschlußvorrichtung eine mittige Aussparung (21) aufweist, um den Einlassungsblock während einer Nutzungsphase der Spritze aufzunehmen, und in ihrer Mittelzone (18) und in der Verlängerung der besagten Aussparung (21) einen vorausgeschnittenen Spalt (19) aufweist, der durch zwei Lippen festgelegt ist, und wobei die Spitze (26) mit einem Ende versehen ist, das entsprechend einer senkrecht zu ihrer Achse liegenden Ebene geschnitten und so angeordnet ist, daß die Lippen beabstandet werden, und der Spalt geöffnet wird, um die Axialleitung (23) mit dem Reservoir (11) in Verbindung zu bringen, daß der Durchmesser der Spitze (26) kleiner als der Durchmesser der Mittelzone (18) der Kolben-Ventil-Verschlußvorrichtung (17) ist,
**dadurch gekennzeichnet,** daß
der Einlassungsblock (25) einen geraden Teil (25b) aufweist, der mit zwei Wülsten (36) und (37) versehen ist, die jeweils auf beiden Seiten der Umfangsrille (35) angeordnet sind, wobei der erste Wulst (36) so angeordnet ist, daß er der Druckkraft der Ausbauchung (38) einen niedrigeren Widerstand bietet als derjenige des zweiten Wulstes (37).

2. Spritze nach Patentanspruch 1,
**dadurch gekennzeichnet,** daß
die Stange (22) einstückig mit einem Fuß (24) ausgeführt ist, der so angeordnet ist, daß er sich gegen eine Innenfläche des entfernten Endes des zylindrischen Hohlkörpers (13) abstützt.

3. Spritze nach Patentanspruch 1,
**dadurch gekennzeichnet,** daß
der Einlassungsblock (25) ein breites Teil (25a) aufweist, dessen Durchmesser etwa gleich dem Innendurchmesser des zylindrischen Hohlkörpers (13) ist, um die Zentrierung der Stange (22) im Inneren des Körpers sicherzustellen.

4. Spritze nach Patentanspruch 1,
**dadurch gekennzeichnet,** daß
der erste Wulst (36) einen Abschnitt unterhalb desjenigen des zweiten Wulstes (37) aufweist.

5. Spritze nach Patentanspruch 1,
**dadurch gekennzeichnet,** daß
das Reservoir (11) einen Außenwulst (30) aufweist, der so angeordnet ist, daß er mit einer Rille (31) zusammenwirkt, die am freien Ende des Körpers (13) angeordnet ist.

6. Spritze nach Patentanspruch 1,
**dadurch gekennzeichnet,** daß
das Reservoir (11) einen Innenwulst (32) aufweist, der so angeordnet ist, daß er mit einer Ausbauchung (33) zusammenwirkt, die am Ende der Nutzung an der Basis der Stange (22) angeordnet ist.

7. Spritze nach Patentanspruch 1,
**dadurch gekennzeichnet,** daß
der vorausgeschnittene Spalt (19) im Verhältnis zur mittigen Aussparung (21) der Kolben-Ventil-Verschlußvorrichtung (17) zentriert ist.
